(19) Europäisches Patentamt
European Patent Office
Office européen des brevets

(11) **EP 0 614 660 B1**

## (12) FASCICULE DE BREVET EUROPEEN

(45) Date de publication et mention
de la délivrance du brevet:
**14.10.1998 Bulletin 1998/42**

(51) Int. Cl.$^6$: **A61K 9/113**, A61K 7/00

(21) Numéro de dépôt: **94400493.6**

(22) Date de dépôt: **08.03.1994**

(54) **Nouvelles émulsions multiples, leur préparation, leur application à la préparation de compositions cosmétiques et ces compositions cosmétiques**

Mehrfachemulsionen, ihre Herstellung, ihre Verwendung zur Herstellung von kosmetischen Zubereitungen und diese kosmetischen Zubereitungen

Multiple emulsions, their preparation, their application in the preparation of cosmetic compositions and cosmetics compositions containing them

(84) Etats contractants désignés:
**AT BE CH DE DK ES FR GB GR IE IT LI LU NL PT SE**

(30) Priorité: **11.03.1993 FR 9302795**

(43) Date de publication de la demande:
**14.09.1994 Bulletin 1994/37**

(73) Titulaire: **The Boots Company PLC Nottingham, Nottinghamshire NG2 3AA (GB)**

(72) Inventeur: **Fructus, Alain F-92400 Courbevoie (FR)**

(74) Mandataire:
**Thacker, Michael Anthony et al The Boots Company plc Group Patents Department, D31, 1 Thane Road West Nottingham NG2 3AA (GB)**

(56) Documents cités:
EP-A- 0 345 075    EP-A- 0 507 693
DE-A- 4 136 699    FR-A- 2 326 914
FR-A- 2 693 466

• PATENT ABSTRACTS OF JAPAN vol. 8, no. 015 (C-206) 21 Janvier 1984 & JP-A-58 183 611 (KANEBO KK) 26 Octobre 1983

**Description**

La présente invention concerne de nouvelles émulsions multiples, leur préparation, leur application à la préparation de compositions cosmétiques et ces compositions cosmétiques.

Une émulsion multiple se réalise, en général, en faisant d'abord une émulsion primaire (par exemple eau/huile) puis en dispersant cette émulsion primaire dans une phase aqueuse à l'aide d'émulsionnants.

EP-A 507693 décrit une émulsion triple du type eau/huile/eau comportant une phase aqueuse externe continue gélifiée et contenant au moins à titre de gélifiants un polymère ou copolymère d'acid acrylique ou methacrylique, associé à un polyglycéryl méthacrylate et contenant des emulsifiants utitisés pour la realisation des emulsions.

DE-A-4136699 décrit une émulsion de type eau/huile/eau comportant une phase aqueuse externe qui peut être gélifée par un polyglycéryl méthacrylate et contenant d'un émulsionnant.

EP-A-345075 décrit une émulsion du type eau/huile/eau comportant une phase aqueuse externe contenant d'émulsionnant et ne contenant pas d'un polyglycéryl méthacrylate.

Ainsi le brevet français BF 2,326,914 décrit une émulsion multiple du type eau/huile/eau, comportant une phase dispersée et un milieu dispersant, caractérisée par le fait que :

-   la phase dispersée est une émulsion comportant une phase eau et une phase huile, ladite phase huile étant obtenue par dissolution d'un émulsifiant soluble dans l'huile,
-   et que le milieu de dispersion est une solution aqueuse contenant un émulsifiant soluble dans l'eau.

Il y a donc nécessairement utilisation d'un émulsionnant à chacune des deux étapes de la préparation d'une telle émulsion multiple et il y a souvent interaction entre les émulsionnants nécessaires pour faire l'émulsion primaire et ceux nécessaires pour l'émulsion secondaire.

Il peut en résulter un choix restreint, des stabilités douteuses (notamment par inversions de phases, passage à une émulsion simple, ou encore déphasage), et ainsi une qualité cosmétique moyenne.

La présente invention a pour objet une émulsion multiple destinée à véhiculer un ou plusieurs principes actifs hydrosolubles ou liposolubles se présentant sous la forme d'une émulsion eau/huile/eau constituée d'une phase dispersée qui est une émulsion du type eau/huile et d'un milieu de dispersion aqueux, caractérisée en ce que le milieu de dispersion aqueux est une phase aqueuse gélifiée.

Dans la présente invention, l'émulsion eau/huile primaire est classique, avec des tensio-actifs habituellement utilisés en cosmétique pour ce type d'émulsion.

Mais ensuite cette émulsion primaire est dispersée dans une phase aqueuse gélifiée avec des gélifiants qui remplacent les émulsionnants. Les gouttelettes d'émulsion primaire sont immobilisées par le réseau souple des gels, elles ne peuvent donc coalescer entre elles, d'où une très grande stabilité.

Comme il n'y a pas d'émulsionnant dans la phase aqueuse externe, il n'y a pas perturbation de l'émulsion primaire par diffusion d'émulsionnants.

Au total on a une émulsion multiple, stable, réalisée avec des matières premières cosmétiques, ce qui permet d'avoir une bonne innocuité et un toucher agréable.

La présente invention comporte également l'incorporation de principes actifs hydrosolubles ou liposolubles dans une telle émulsion multiple.

Les buts recherchés d'une telle émulsion multiple selon l'invention sont notamment de séparer des principes actifs hydrosolubles incompatibles en les incorporant dans 2 phases aqueuses séparées par une phase grasse, ce qui permet notamment de préparer des compositions cosmétiques pouvant réaliser sur la peau un relargage progressif des actifs car les structures de l'émulsion multiple se détruisent plus lentement que celles d'une émulsion simple et ainsi, d'augmenter la pénétration de principes actifs car la structure interne des émulsions multiples et notamment triples est dans certains cas, semblable à certaines structures qui sont les fonctions barrières de la peau.

La présente invention a plus précisément pour objet une émulsion multiple telle que décrite ci-dessus, caractérisée en ce que le ou les gélifiants contenus dans la phase aqueuse de dispersion sont choisis parmi :

-   une solution à base de polyglycéryl méthacrylate,
-   des carbomers,
-   des carbomers modifiés

La présente invention a particulièrement pour objet une émulsion multiple telle que décrite ci-dessus, caractérisée en ce que le gélifiant contenu dans la phase aqueuse de dispersion est constitué d'une solution à base de polyglycéryl méthacrylate, de carbomers et de carbomers modifiés.

La solution à base de polyglycéryl méthacrylate peut être du Lubragel® et les carbomers modifiés du Pémulen® TR1 et TR2.

A titre d'illustration d'une telle utilisation de tels gélifiants utilisés seuls ou en mélanges, mais sans toutefois limiter les quantités utilisables de ces gélifiants, les exemples suivants peuvent être donnés dans lesquels les pourcentages préférés sont exprimés par rapport à la formule complète finale :

- Lubragel® : 1 à 30 %,
- Carbomers : 0,01 à 2 %,
- Carbomers modifiés (Pemulen® TR1 et TR2) : 0,04 à 2 %,

Parmi les gélifiants préférés de l'invention, on peut citer par exemple et de façon non limitative, le mélange constitué de 5 à 15 % de LUBRAGEL®, 0,05 à 0,3 % de carbomers et 0,1 à 0,8 % de carbomers modifiés.

La présente invention a particulièrement pour objet une émulsion multiple, telle que décrite ci-dessus, caractérisée en ce que un ou plusieurs principes actifs hydrosolubles sont incorporés dans la phase aqueuse de l'émulsion eau/huile et/ou dans la phase aqueuse de dispersion.

La présente invention a également pour objet une émulsion multiple, telle que décrite ci-dessus, caractérisée en ce que un ou plusieurs principes actifs liposolubles sont incorporés dans la phase grasse de l'émulsion eau/huile.

La présente invention a précisément pour objet une émulsion multiple, telle que décrite ci-dessus, caractérisée en ce que les principes actifs hydrosolubles sont choisis parmi le lactate de sodium, des extraits de biolysat hafnia, des extraits de biolysat de Klebsellia pneumoniae et des filtres solaires hydrosolubles.

De façon préférentielle mais sans toutefois limiter l'invention, les principes actifs hydrosolubles peuvent être choisis parmi :

- l'acide 2-phényl benzimidazol 5-sulfonique neutralisé,
- l'acide 2-hydroxy 4-méthoxybenzophénone 5-sulfonique neutralisé,
- l'acide ascorbique, le benzoate de caféine, l'acide phytique, l'acide mucique, les hydrolysats de protéines végétales, le polyglucan,
- l'extrait de mimosa du mexique, le chitosan, le sérum d'animaux marins,
- l'extrait d'hirudine, l'extrait de méristem, les oligomères procyanodoliques, les extraits de levures, le panthénol, l'extrait de centella asiatica et l'acide glycyrrhétinique.

A titre d'illustration d'une telle utilisation de tels principes actifs hydrosolubles, mais sans toutefois limiter les quantités utilisables de ces principes actifs, les exemples suivants peuvent être donnés dans lesquels les pourcentages préférés sont exprimés par rapport à la formule complète finale :

- Acide 2-phényl benzimidazol 5-sulfonique neutralisé : 0,5 à 8 %,
- Acide 2-hydroxy 4-méthoxybenzophénone 5-sulfonique neutralisé : 0,5 à 5 %,
- Acide ascorbique : 0,5 à 10 %, benzoate de caféine : 0,1 à 5 %, acide phytique : 0,1 à 5 %, acide mucique : 0,1 à 5 %, hydrolysats de protéines végétales : 0,1 à 10 %, polyglucan : 0,1 à 5 %,
- Extrait de mimosa du mexique : 0,5 à 20 %, chitosan : 0,5 à 20 %, sérum d'animaux marins : 0,1 à 3 %,
- Extrait d'hirudine : 0,5 à 10 %, extrait de méristem : 0,1 à 5 %, oligomères procyanodoliques : 0,05 à 3 %, extraits de levures : 0,05 à 3 %, panthénol : 0,05 à 5 %, extrait de centella asiatica : 0,05 à 3 %, l'acide glycyrrhétinique : 0,05 à 2 %.

La présente invention a ainsi également pour objet une émulsion multiple, telle que décrite ci-dessus, caractérisée en ce que les principes actifs liposolubles sont choisis parmi le palmitate de vitamine A, des filtres solaires liposolubles, des insaponifiables d'origine végétale, des huiles d'origine végétale, de l'acétate de tocophérols, des tocophérols naturels, du farnesol, des céramides naturelles ou de synthèse.

De façon préférentielle mais sans toutefois limiter l'invention, on peut indiquer que :

- les filtres solaires liposolubles peuvent être choisis parmi l'octyl méthoxycinnamate, l'isoamyl méthoxycinnamate, l'octyl diméthyl paba, l'octyl salicylate, le butyl méthoxydibenzoyl méthane, le benzophénone 3, l'octyl triazone, le 4-polyéthoxy aminobenzoate d'éthyle, l'isopropyle 4-dibenzoyl méthane,
- les insaponifiables de végétaux peuvent être choisis parmi des insaponifiables de maïs, de karité, de soja et d'avocat,
- les huiles d'origine végétale peuvent être péroxydées ou non et notamment choisies parmi un mélange huileux renfermant de l'acide xyménique, de l'extrait essentiel d'huile de sésame, de l'huile de maïs peroxydée, de l'huile d'onagre, de l'huile d'onagre peroxydée, de l'huile de bourrache et de l'huile de bourrache peroxydée.

Le mélange huileux contenant de l'acide xyménique peut être du Xyménoil® qui renferme 50 % de cet acide.

A titre d'illustration d'une telle utilisation de tels principes actifs liposolubles, mais sans toutefois limiter les quantités utilisables de ces principes actifs, les exemples suivants peuvent être donnés dans lesquels les pourcentages préférés sont exprimés par rapport à la formule complète finale.

- Palmitate de vitamine A : de 500 à 10 000 UI/g.
- Filtres solaires liposolubles : octyl méthoxycinnamate : 0,5 à 10 %, isoamyl méthoxycinnamate : 0,5 à 10 %, octyl diméthyl paba : 0,5 à 8 %, octyl salicylate : 0,5 à 5 %, butyl méthoxydibenzoyl méthane : 0,5 à 5 %, benzophénone 3 : 0,5 à 10 %, octyl triazone : 0,5 à 5 %, 4-polyéthoxy aminobenzoate d'éthyle : 0,5 à 10 %, isopropyle-4-dibenzoyl méthane : 0,5 à 5 %.
- Insaponifiables de maïs, de karité, de soja ou d'avocat : 0,1 à 3 %.
- Ximénoil<sup>®</sup> : 0,1 à 5 %, extrait essentiel d'huile de sésame: 0,1 à 4 %, huile de maïs péroxydée : 0,1 à 10 %, huile d'onagre : 0,1 à 10 %, huile d'onagre péroxydée : 0,1 à 10 %, huile de bourrache : 0,1 à 10 %, huile de bourrache péroxydée: 0,1 à 10 %, acétate de tocophérols : 0,05 à 7 %, tocophérols naturels : 0,05 à 5 %, farnesol : 0,05 à 5 %, céramides naturelles ou de synthèse : 0,01 à 10 %.

La présente invention a plus particulièrement pour objet une émulsion multiple, telle que décrite ci-dessus, caractérisée en ce que les volumes des différentes phases sont tels que :

- la phase aqueuse interne représente 20 à 30 % de la composition totale,
- la phase grasse représente 10 à 20 % de la composition totale,
- et la phase aqueuse externe représente 50 à 60 % de la composition totale.

La présente invention a également pour objet un procédé de préparation d'une émulsion multiple, comprenant :

- la préparation de la phase dispersée en mélangeant, de préférence en chauffant, la phase aqueuse et la phase grasse sous forte agitation et
- le mélange de ladite phase dispersée avec la phase aqueuse externe, de préférence à température ordinaire, sous faible agitation jusqu'à formation de l'émulsion multiple, procédé caractérisé en ce que la phase aqueuse externe est gélifiée et ne contient pas d'émulsionnant.

De façon préférentielle, l'émulsion primaire est réalisée en introduisant la phase aqueuse portée à 80°C dans la phase huileuse également à 80°C sous agitation élevée.

La deuxième étape consiste à mélanger l'émulsion primaire et la phase aqueuse externe gélifiée à température ordinaire, sous faible agitation jusqu'à la formation de l'émulsion multiple.

La présente invention a particulièrement pour objet un procédé, tel que décrit ci-dessus, de préparation d'une émulsion multiple, telle que décrite ci-dessus, caractérisé en ce que le gélifiant que l'on utilise pour préparer la phase aqueuse externe est choisi parmi :

- une solution à base de polyglycéryl méthacrylate,
- la gomme xanthane,
- des polysaccharides,
- des carbomers,
- des carbomers modifiés,
- de l'hydroxy éthyl cellulose et hydroxy éthyl cellulose modifiée.

Des exemples d'utilisation de tels gélifiants ont été indiqués ci-dessus.

La présente invention a également pour objet une composition cosmétique, caractérisée en ce qu'elle renferme une émulsion multiple, telle que décrite ci-dessus et le cas échéant un ou plusieurs excipients usuels inclus dans l'émulsion multiple, ainsi qu'un ou plusieurs principes actifs appropriés.

Parmi les excipients usuels, on peut citer notamment les excipients de la phase grasse tels que par exemple des huiles végétales comme l'huile de jojoba, d'olive, d'avocat, de macadamia, des huiles minérales comme l'huile de vaseline, des hydrocarbures comme le dioxtyl cyclohexane ou l'isohexadécane, des esters comme le palmitate de cétyle ou l'isostéarate d'isocétyle, des cires naturelles comme la cire d'abeille, de carnauba, de candellila, des triglycérides semi synthétiques comme les triglycérides capryliques ou capriques, des alcools gras comme l'alcool cétylique ou stéarylique, des esters du propylène glycol comme le monostéarate de propylène glycol.

On peut citer également comme additifs dans la phase aqueuse des antiseptiques, des conservateurs comme les parabens, le bronopol, le biosol, des humectants comme la glycérine, le propylène glycol, le sorbitol, le polyoxyéthylène glycol 400 ainsi que des colorants.

Les compositions cosmétiques selon l'invention peuvent se présenter sous toutes les formes utilisées en dermatologie, plus particulièrement celles pouvant être administrées par voie topique.

Les compositions cosmétiques peuvent être présentées comme des produits usuels des soins de la peau, par exemple sous forme de crèmes hydratantes, crèmes écran total, crèmes de jour, crèmes de nuit, masques ou comme des produits de maquillage, par exemple, fond de teint et crème teintée, ou comme des produits de démaquillage ou encore comme des produits d'hygiène.

De telles compositions peuvent être liquides ou pâteuses : parmi de telles présentations peuvent être citées par exemple, les crèmes : crème hydratante, crème de jour, crème de nuit, pommades, masques, crème de maquillage, laits, ainsi que les lotions et notamment les lotions solaires.

De telles présentations indiquées ci-dessus sont conditionnées, selon le cas, en pots ou en tubes, en flacon de verre ou de plastique ou éventuellement en flacon doseur ou encore en ampoules et sont préparées selon les méthodes usuelles.

La présente invention a notamment pour objet une composition cosmétique destinée en particulier à la protection solaire, caractérisée en ce qu'elle renferme une émulsion multiple, telle que décrite ci-dessus, le cas échéant un ou plusieurs excipients usuels inclus dans l'émulsion multiple, ainsi qu'un ou plusieurs principes actifs appropriés.

La présente invention a particulièrement pour objet une composition cosmétique, telle que décrite ci-dessus, caractérisée en ce qu'elle se présente sous forme d'une lotion solaire.

L'invention concerne également de nouvelles compositions cosmétiques, telles que définies ci-dessus, destinées notamment au soin des comédons, caractérisée en ce qu'elle renferme une émulsion multiple telle que décrite ci-dessus, le cas échéant un ou plusieurs excipients usuels ainsi qu'un ou plusieurs principes actifs appropriés.

La présente invention a encore également pour objet une composition cosmétique telle que définie ci-dessus destinée aux peaux acnéiques, aux peaux déshydratées, aux peaux lésées, aux peaux ridées, caractérisée en ce qu'elle renferme une émulsion multiple telle que définie ci-dessus, le cas échéant un ou plusieurs excipients usuels, ainsi qu'un ou plusieurs principes actifs appropriés.

Les compositions cosmétiques peuvent être préparées en utilisant les ingrédients indiqués ci-dessus.

La présente invention a aussi pour objet l'application d'émulsion multiple, telle que décrite ci-dessus, à la préparation de composition cosmétique telle que définie ci-dessus.

Les différentes formes cosmétiques mentionnées ci-dessus peuvent être obtenues selon les méthodes usuelles utilisées dans ces domaines.

Les exemples donnés ci-après montreront de manière plus précise, les caractéristiques et les avantages apportés par la présente invention. Il est évident que diverses modifications peuvent être apportées à ces exemples sans cependant sortir de l'invention.

Exemples de préparation d'émulsion multiple selon l'invention sans principe actif

**EXEMPLE 1 :**

**a) On prépare une émulsion primaire eau/huile de la façon suivante :**

On chauffe à 80°C la phase aqueuse suivante : dite phase aqueuse interne :

| | |
|---|---|
| - eau déminéralisée | 26,52 g |
| - méthyl paraben | 0,1 g |
| - sulfate de magnésium | 0,28 g |
| - glycérine 30°B | 0,8 g |
| - O-cymen-5-ol | 0,04 g |

On chauffe séparément à 80°C également la phase grasse suivante :

| | |
|---|---|
| - glycéryl isostéarate | 2,0 g |

(suite)

| | |
|---|---|
| - huile de ricin hydrogénée polyéthoxylée (7 moles) | 0,2 g |
| - triglycérides capriques/capryliques | 8,2 g |
| - propyl paraben | 0,06 g |
| - huile de silicone volatile | 1,6 g |

On disperse la phase aqueuse dans la phase grasse à 80°C en agitant vigoureusement 5 minutes. Puis on refroidit lentement jusqu'à 25°C.

**b) On prépare l'émulsion multiple de la façon suivante :**

On disperse ensuite cette émulsion primaire, obtenue en a), dans la phase aqueuse suivante, dite phase aqueuse externe, en mélangeant doucement à température ambiante :

| | | |
|---|---|---|
| - eau déminéralisée | QS | 100,0 g |
| - Lubragel MS ® | | 15,0 g |
| - Pemulen TR1 ® | | 0,6 g |
| - Carbopol 980 ® | | 0,03 g |
| - Tétrasodium EDTA | | 0,054 g |
| - Méthyl paraben | | 0,216 g |
| - Imidazolidinyl urée | | 0,216 g |
| - Soude pure | | 0,125 g |
| - Parfum | | 0,2 g |

**Caractérisation par étude rhéologique**

L'étude rhéologique est effectuée à l'aide d'un rhéomètre CSL 100 à contrainte imposée (RHEO, 91 Champlan) à 25±1°C. La géométrie du cisaillement utilisée est une géométrie cône/plateau (diamètre = 4 cm, angle de cône = 2°) et permet de faire les analyses en balayage contraintes de 0,6 Pa à 600 Pa. Le capteur de déplacement a une résolution de $10^{-5}$ rd. Les résultats sont analysés avec le logiciel Carri50.
Deux type d'études sont réalisées :

**1 - Analyse viscoélastique oscillatoire (dynamique)**

Cette analyse est effectuée de façon à fournir une signature très précise et caracctéristique de la structure "au repos" des échantillons.
Dans ce type d'analyse, l'échantillon est soumis à un cisaillement sinusoïdal de pulsation $\omega = 2\pi N$ (N : fréquence de cisaillement), qui est défini par les expressions suivantes de la contrainte et de la déformation de cisaillement.

- Contrainte de cisaillement $\tau(t) = \tau_0 \cos\omega t$
- Déformation de cisaillement $\varepsilon(t) = \varepsilon_0 \cos(\omega t + \delta)$

Ces expériences permettent de définir un certain nombre de grandeurs rhéologiques caractéristiques de l'échantillon.

- Module de cisaillement $G^* = \tau_0/\varepsilon_0$
- Déphasage (contrainte/déformation) $\delta$
- Module de perte $G'' = G^*\sin\delta$

Au cours de cette expérience, on procède à un balayage en contrainte à fréquence fixée, au cours duquel on enregistre l'évolution de $G^*$, $\delta$, $G''$. On obtient une zone plateau caractéristique d'une structure non modifiée (structure au repos), pour des valeurs de la contrainte inférieure à une certaine valeur critique $(\tau_0)_c$ ou contrainte pour laquelle $G''$ représente un maximum.

Les grandeurs rhéologiques dynamiques retenues comme grandeurs caractéristique de la structure sont :

- la valeur moyenne de $G^*$ calculée au niveau du palier,
- la valeur moyenne de $\delta$ calculée au niveau du palier,
- la valeur de la contrainte critique $(\tau_0)_c$
- la valeur de la déformation critique $(\varepsilon_0)_c$.

On obtient les valeurs indiquées dans le tableau ci-dessous pour l'émulsion multiple de l'exemple 1.

| | |
|---|---|
| $G^*$ (Pa) | 155 |
| $\delta$ (deg) | 10 |
| $\tau_c$ (Pa) | 72 |
| $\varepsilon_c$ | 0,78 |

**2 - Analyse en régime d'écoulement permanent**

Ce type de test est destiné à fournir des informations sur le rôle du cisaillement dans une éventuelle destructuration et sur son caractère plus ou moins réversible. Cette expérience est conduite dans des conditions de cisaillement qui restituent celles qui seraient éventuellement mises en oeuvre lors d'une application sur la peau (vitesse maximale de cisaillement $\dot{\varepsilon}$ max = $10^3$ $S^{-1}$). Pour ce type d'analyse on procède à des tests d'écoulement permanent au cours desquels l'échantillon sera cisaillé selon des cycles de contraintes successivement croissantes (montée), constantes (palier) et décroissantes (descente).

Cette succession de cycles de contraintes est appliquée sur un échantillon selon le protocole :

a) Cycle de cisaillement sur un échantillon "neuf" n'ayant subi aucun précisaillement,
b) Cycle de cisaillement sur le même échantillon après un repos de 1 heure,
c) Cycle de cisaillement sur le même échantillon après un précisaillement de 1 heure sous vitesse de $10^3$ $S^{-1}$.

Le traitement quantitatif du rhéogramme est effectué en cherchant le meilleur ajustement qui est le plus souvent fourni par le modèle de CROSS.

Cette analyse en régime d'écoulement permanent est complétée par les analyses rhéologiques oscillatoires qui ont été effectuées sur le même échantillon, avant ou après chaque cycle de cisaillement. Plus précisément, quatre balayages en contrainte à fréquence et température fixées (N=1Hz, t=20°C) ont été réalisés, selon la sucession suivante :

a' : sur l'échantillon "frais", avant le cycle a,
b' : immédiatement après le cycle a,
c' : immédiatement après le cycle b,
d' : immédiatement après le cycle c.

On obtient donc pour le produit de l'émulsion multiple de l'exemple 1, les résultats d'analyses rhéologiques oscillatoires (cycles a', b', c' et d') qui ont été effectuées sur le même échantillon avant et après chaque cycle de cisaillement (cycles a, b, c) et les valeurs moyennes de $G^*$ indiquées dans le tableau ci-dessous :

| Balayage | $G^*$(Pa) |
|---|---|
| Cycle a' | 133 |
| Cycle b' | 111 |

(suite)

| Balayage | G*(Pa) |
|----------|--------|
| Cycle c' | 105 |
| Cycle d' | 117 |

Conclusion :

On observe une variation relative de G* qui est très faible et du même ordre de grandeur que les variations relatives des paramètres de la loi de Cross. Le cisaillement exerce une influence plutôt négligeable sur les échantillons (dans l'intervalle des contraintes utilisées). Il s'agit là d'une propriété intéressante sur le plan de stabilité de l'émulsion multiple réalisée de l'exemple 1.

**EXEMPLE 2 :**

**a) On prépare une émulsion primaire eau/huile de la façon suivante :**

On chauffe à 80°C la phase aqueuse suivante : dite phase aqueuse interne :

| - eau déminéralisée | 26,52 g |
|---------------------|---------|
| - méthyl paraben | 0,1 g |
| - sulfate de magnésium | 0,28 g |
| - glycérine 30°B | 0,8 g |
| - O-cymen-5-ol | 0,04 g |

On chauffe séparément à 80°C également la phase grasse suivante :

| - glycéryl isostéarate | 2,0 g |
|------------------------|-------|
| - PEG-7 hydrogenated Castor oil | 0,2 g |
| - triglycérides capriques/capryliques | 8,2 g |
| - propyl paraben | 0,06 g |
| - mineral oil/quaternium 18 hectorite/propylène carbonate | 0,2 g |
| - cyclométhicone | 1,6 g |

On disperse la phase aqueuse dans la phase grasse à 80°C en agitant vigoureusement 5 minutes. Puis on refroidit lentement jusqu'à 25°C.

**b) On prépare l'émulsion multiple de la façon suivante :**

On disperse ensuite cette émulsion primaire, obtenue en a), dans la phase aqueuse suivante, dite phase aqueuse externe, en mélangeant doucement à température ambiante :

| - polyglycéryl méthacrylate/propylène glycol | 15,0 g |
|----------------------------------------------|--------|
| - acrylates/C10-30 alkyl acrylate crosspolymer | 0,6 g |

(suite)

| | |
|---|---|
| - carbomer 980 | 0,03 g |
| - tétrasodium EDTA | 0,054 g |
| - méthylparaban | 0,216 g |
| - imidazolidinyl urée | 0,216 g |
| - hydroxyde de sodium | 0,125 g |
| - eau | QS      100,0 g |

Exemples de préparation d'émulsion multiple selon l'invention avec principe actif

**EXEMPLE 3 :**

**a) On prépare une émulsion primaire eau/huile de la façon suivante** :

On chauffe à 80°C la phase aqueuse suivante : dite phase aqueuse interne :

| | |
|---|---|
| - eau déminéralisée | 26,52 g |
| - méthyl paraben | 0,1 g |
| - sulfate de magnésium | 0,28 g |
| - glycérine 30°B | 0,8 g |
| - O-cymen-5-ol | 0,04 g |

On chauffe séparément à 80°C également la phase grasse suivante :

| | |
|---|---|
| - glycéryl isostéarate | 2,0 g |
| - huile de ricin hydrogénée polyéthoxylée (7 moles) | 0,2 g |
| - triglycérides capriques/capryliques | 8,2 g |
| - propyl paraben | 0,06 g |
| - huile de silicone volatile | 1,6 g |

On disperse la phase aqueuse dans la phase grasse à 80°C en agitant vigoureusement 5 minutes. Puis on refroidit lentement jusqu'à 25°C.

**b) On prépare l'émulsion multiple de la façon suivante :**

On disperse ensuite cette émulsion primaire, obtenue en a), dans la phase aqueuse suivante, dite phase aqueuse externe, en mélangeant doucement à température ambiante :

| | |
|---|---|
| - eau déminéralisée | QS      100,0 g |
| - Lubragel MS ® | 15,0 g |
| - Pemulen TR1 ® | 0,6 g |

9

(suite)

| | |
|---|---|
| - Carbopol 980 ® | 0,03 g |
| - Biolysat Hafnia | 0,03 g |
| - Tétrasodium EDTA | 0,054 g |
| - Méthyl paraben | 0,216 g |
| - Imidazolidinyl urée | 0,216 g |
| - Soude pure | 0,125 g |
| - Parfum | 0,2 g |

## EXEMPLE 4 :

Le produit de l'exemple 4 a été préparé suivant le même procédé que pour préparer le produit de l'exemple 3 en incorporant comme principes actifs des filtres solaires hydrosolubles (3,2 g) dans l'émulsion primaire eau/huile à la place du palmitate de vitamine A et des filtres solaires liposolubles (2,8 g) dans la préparation de l'émulsion multiple à la place du Biolysat Hafnia.

## EXEMPLE 5 : Emulsion triple E/H/E

On réalise à 80°C, l'émulsion primaire E/H suivante que l'on fait ensuite refroidir lentement à température ambiante:

| | |
|---|---|
| - glycéryl isostéarate | 2,0 g |
| - huile de ricin hydrogénée polyoxyéthylène 7OE | 0,2 g |
| - caprylic caprique triglycérides | 8,2 g |
| - parahydroxybenzoate de propyle | 0,06 g |
| - huile de silicone volatile | 1,6 g |
| - palmitate de vit. A à 1,7 M UI/g | 0,12 g |
| - eau déminéralisée | 26,52 g |
| - parahydroxybenzoate de méthyle | 0,1 g |
| - sulfate de magnésium | 0,28 g |
| - glycérine 30° codex | 0,8 g |
| - O-cymen 5-ol | 0,04 g |

On réalise ensuite l'émulsion multiple en dispersant lentement à température ambiante l'émulsion primaire dans la phase aqueuse suivante :

| | |
|---|---|
| - Lubrajel MS ® | 15,0 g |
| - polymère carboxyvinylique | 0,03 g |
| - tétrasodium EDTA | 0,054 g |
| - parahydroxybenzoate de méthyle | 0,216 g |
| - imidazolidinyl urée | 0,216 g |
| - hydroxyde de sodium pur | 0,125 g |

(suite)

| | |
|---|---|
| - biolysat hafnia | 0,02 g |
| - eau déminéralisée | QS    100,0 g |

**EXEMPLE 6** :

Le produit de l'exemple 6 a été préparé suivant le même procédé que pour préparer le produit de l'exemple 3 en incorporant comme principe actif du lactate de sodium hydratant (6 g) à la place du Biolysat Hafnia.

Ainsi plusieurs types de principes actifs ont été incorporés dans une émulsion multiple, selon les préparations indiquées ci-dessus aux exemples 3, 4, 5 et 6, et ainsi que résumé dans le tableau suivant :

| | Phase aqueuse interne | Phase grasse | Phase aqueuse externe |
|---|---|---|---|
| 3 | Biolysat hafnia | - | - |
| 4 | Filtre solaire hydrosoluble | Filtre solaire liposoluble | - |
| 5 | - | Palmitate de vitamine A | - |
| 6 | Lactate de sodium hydratant | - | - |

**Exemple 7 :**

a) On prépare une émulsion primaire eau/huile de la façon suivante :

| | |
|---|---|
| - eau déminéralisée | 35,5 g |
| - méthyl paraben | 0,1 g |
| - sulfate de mégnésium | 0,28 g |
| - Glycérine 30°B | 2,0 g |
| - O-cymen-5-ol | 0,04 g |
| - Extrait de Biolysat de Klebsellia pneumoniae | 0,005g |

On chauffe séparément à 80°C également la phase grasse suivante :

| | |
|---|---|
| - glycéryl isostéarate | 2 g |
| - cétyl diméthicone copolyol (ABIL FM 90 [R]) | 0,4 g |
| - triglycérides 5545 | 1,0 g |
| - huile de silicone volatile | 2,0 g |
| - huile de bourrache | 2,0 g |

on disperse la phase aqueuse dans la phase grasse à 80°C en agitant vigoureusement 5 minutes. Puis on refroidit lentement jusqu'à 25°C.

b) On prépare l'émulsion multiple de la façon suivante :

On disperse ensuite cette émulsion primaire obtenue en a) dans la phase aqueuse suivante, dite phase aqueuse externe, en mélangeant doucement à température ambiante :

| | | |
|---|---|---|
| - lubragel MS (R) | | 8 g |
| - hydroxyéthyl cellulose modifiée (AMERCEL HM 1500 $^R$) | | 0,6 g |
| - tétrasodium EDTA | | 0,054g |
| - méthyl paraben | | 0,216g |
| - imidazolidinyl urée | | 0,125g |
| - parfum | | 0,2 g |
| - extrait glycolique de mimosa du mexique | | 5,0 g |
| - eau déminéralisée | QS | 100 g |

## ETUDE CLINIQUE

**I - Test d'inocuité et de mesure d'indice de protection solaire**

1 - Protocole :

a) <u>Irritation primaire cutanée</u>

Le produit étudié est appliqué tel quel sous tampon occlusif sur les flancs normaux et incisés des 6 lapins à la dose de 0,5 ml selon le protocole suivant :

- les 6 lapins sont tondus 24 heures avant l'essai au niveau du dos et des flancs ;
- à l'aide d'un vaccinostyle, 3 scarifications parallèles (d'environ 3 cm de long, espacées de 0,5 cm) sont effectuées sur le flanc droit de chaque animal ; l'autre flanc est maintenu intact ;
- le produit est déposé sur 2 pièces carrées de gase hydrophile Codex à 8 épaisseurs de 2,5 cm de côté ;
- une pièce de gase est appliquée sur chacun des flancs ;
- le tronc de l'animal est entouré avec une bande extensible de 10 cm de large ;
- les lapins sont remis dans leur cage pendant 24 heures au terme desquelles le pansement et les 2 pièces de gase sont enlevés.

b) <u>Irritation oculaire</u>

Après avoir vérifié que les 6 lapins avaient des yeux sains et sans défauts,

- le produit est instillé tel quel à raison de 0,1 ml dans la paupière inférieure éversée de l'oeil droit de chaque lapin ;
- les paupières sont maintenues fermées pendant 10 secondes environ pour éviter la perte de produit et favoriser le contact ;
- après un temps de contention d'une heure, les animaux sont replacés dans leurs cages individuelles.

2 - Résultats :

a) <u>Irritation primaire cutanée</u>

Dans les conditions expérimentales adoptées, le produit de l'exemple 4 appliqué tel quel sur la peau, a provoqué l'apparation d'une légère réaction d'irritation (érythème) sur la peau intacte et scarifiée respectivement de 3 à 6 animaux. Ces phénomènes sont apparus presque totalement réversibles 48 heures plus tard.
Aucune modification structurelle notable n'a été observée.
L'indice d'irritation primaire cutané calculé selon la méthode officielle est égal à 0,5.
Cet indice conduit à classe le produit de l'exemple 4 parmi les substances non irritantes pour la peau.

b) <u>Irritation oculaire</u>

Dans les conditions expérimentales adoptées l'instillation du produit de l'exemple 4, tel quel au niveau de l'oeil, a provoqué l'apparition d'une légère réaction d'irritation localisée au niveau de la conjonctive des 6 animaux (énanthème, chémosis et larmoiements abondants) et de l'iris de 4 animaux (congestion). Ces phénomènes sont apparus réversibles en 24 heures pour l'iris et en 72 heures pour la conjonctive.

L'indice d'irritation oculaire maximum (IOMa) est obtenu 1 heure après l'instillation. Il est égal à 15,3.

Les indices d'irritation oculaire moyens sont respectivement : 3,7 à 24 heures, 1 à 48 heures et 0 à partir de 72 heures.

Ces indices conduisent à classer le produit de l'exemple 4 parmi les substances très faiblement irritantes pour l'oeil.

Par comparaison une crème sans émulsionnant contenant les mêmes filtres solaires aux mêmes doses a eu comme résultat 1,2 faiblement irritant en irritation primaire cutanée et très faiblement irritant en irritation oculaire.

<u>Conclusion</u> :

L'émulsion multiple se révèle donc particulièrement bien tolérée par la peau.

**II - Test de mesure de l'indice de protection solaire.**

1 - Protocole expérimental :

<u>Détermination de la source de rayonnement UVB</u>

L'appareil utilisé est une lampe xénon de 150 W comme source de rayonnement UVB/UVA.

Le rayonnement UV est conduit par 6 filtres optiques munies à leur extrémité de 2 fibres de 1 mm d'épaisseur, WG 320 et UG 11, permettant l'obtention d'un spectre continu de longueurs d'onde comprises entre 290 et 390 nm. Chaque spot a une forme sphérique de 8 mm de diamètre.

L'énergie émise par chaque fibre est réglée au moyen d'un écran et les 6 fibres délivrent des énergies dont les valeurs croissent selon une progression géométrique de raison 1,25. Les valeurs affichées par l'appareil sont exprimées en DEM/min.

<u>Contrôle médical préalable</u>

Avant toute application de produit, chaque sujet est soumis à un contrôle médical destiné à préciser son phototype et à vérifier l'absence de toute dermatose ou de tout traitement local à visée thérapeutique.

<u>Zones expérimentales</u>

La recherche de la dose érythémateuse minimale est effectuée pour chaque sujet sur la peau de la région dorsale.

Les produits à tester sont appliqués sur la zone dorsolombaire de part et d'autre de la colonne vertébrale.

<u>Quantités de produits appliquées</u>

Les produits sont appliqués sur une surface de peau de 35 cm$^2$.

Il est appliqué 2 mg de produit par cm$^2$ de peau sur la surface ainsi délimitée et parfaitement repérée.

<u>Recherche de la dose érythémateuse minimale (DEM) sur la peau non protégée</u>

La DEM est recherchée pour chacun des sujets avant toute application de produit.

Elle correspond à la première dose énergétique d'UVB susceptible d'induire un érythème "non ambigu" de la peau non protégée, couvrant la totalité ou la plus grande partie de la zone irradiée.

Cette évaluation est faite entre 20 et 24 heures après l'exposition des sujets à l'irradiation, sous un éclairage type "lumière de jour".

<u>Essai proprement dit</u>

Vingt minutes environ après la stabilisation thermique de la lampe, l'exposition est effectuée environ 15 minutes après application du produit.

Pour chacun des produits les 6 doses de rayonnement UV appliquées sont déterminées selon une progression géométrique de raison 1,25.

20 à 24 heures après cette exposition, on procède à une évaluation de l'érythème cutané sous un éclairage de type "lumière du jour". Est retenue la première dose énergétique d'UVB induisant un érythème "non ambigu" de la peau protégée, couvrant la totalité ou la plus grande partie de la zone irradiée.

Expression des résultats

Les indices de protection moyens sont calculés à partir des coefficients de protection individuels Qi correspondant pour chaque sujet au rapport entre la DEM obtenue sur la peau protégée et la DEM obtenue sur la peau non protégée (méthode de Schultze).

L'Indice de Protection Solaire du produit est calculé par la méthode arithmétique et exprimé par la moyenne et l'écart type obtenus à partir des résultats individuels.

En fonction de l'indice obtenu, le produit est classé dans l'une des catégories suivantes :

- Protection minimale pour les SPF compris entre 2 et 4,
- Protection moyenne pour les SPF compris entre 4 et 6,
- Protection haute pour les SPF compris entre 6 et 8,
- Protection maximale pour les SPF compris entre 8 et 15,
- Protection ultra pour les SPF supérieurs à 15.

2 - Résultats

Recherche de la DEM avant application des produits

L'appareil délivre 6 énergies de rayonnement U.V. qui croissent selon une progression géométrique de raison 1,25 et sont exprimées en DEM/min soit :

$$E = 0{,}66 - 0{,}82 - 1{,}02 - 1{,}28 - 1{,}60 - 2{,}00$$

Le temps d'exposition t1 (sec.) est choisi pour chacun des sujets en fonction de son phototype.
L'énergie E1 pour laquelle on obtient un érythème net est notée en DEM/min.
La DEM initiale D1 pour chaque sujet est calculée selon la formule :

$$D1 = \frac{t1 \times E1}{60}$$

Evaluation des indices de protection solaire

L'appareil comme précédemment délivre 6 énergies de rayonnement U.V. qui croissent selon une progression géométrique de raison 1,25 et sont exprimées en DEM/min soit :

$$E = 0{,}66 - 0{,}82 - 1{,}02 - 1{,}28 - 1{,}60 - 2{,}00$$

La détermination du temps d'exposition t2 (sec.) se fait en fonction de la DEM initiale D1 de chaque sujet et du coefficient de protection maximum supposé du produit à tester (soit n).
Dans le cas présent :

n =     7 pour le standard
        11 pour le produit de l'exemple 4.

Le sujet est exposé à une énergie maximale de :

x D1 =     7 x D1 pour le standard
           11 x D1 pour le produit de l'exemple 4.

L'énergie maximale donnée par la lampe étant fixée à 2 DEM/min, pour obtenir n x D1 il faut un temps d'exposition :

$$t2 = \frac{n \times D1 \text{ min}}{2} \text{ ou } 30 \text{ nD1 sec.}$$

soit 210 D1 sec. pour le standard

soit 330 D1 sec. pour le produit de l'exemple 4.

L'énergie E2 pour laquelle on obtient un érythème net est notée en DEM/min.

La DEM D2 après application des produits pour chaque sujet est calculée selon la formule :

$$D2 = \frac{t2 \times E2}{60}$$

L'indice de protection solaire approché est calculé de la façon suivante :

$$I.P.S. = \frac{D2}{D1}$$

Dans les conditions de l'essai :

- l'indice de protection solaire moyen approché du standard défini par la FDA, est égal à : $3,4 \pm 0,8$. Le test est validé puisque le coefficient de protection solaire obtenu avec le standard est situé à l'intérieur des limites acceptées pour la méthode (entre 3,1 et 5,3).
- l'indice de protection solaire moyen du produit de l'exemple 4, testé dans les mêmes conditionns, est égal à : $6,4 \pm 0,9$.
- ce même indice de protection solaire a été déterminé pour le produit de l'exemple 4, 2 heures après application, pour tester la rémanence du produit. Dans ces conditions l'indice de protection solaire est égal à : $6,1 \pm 1,2$.

Conclusion :

Ces résultats montrent donc l'efficacité en terme de protection solaire du produit de l'exemple 4.

Le test d'innocuité décrit ci-dessus a été réalisé pour le produit de l'exemple 4 et ce produit a été classé très faiblement irritant en irritation oculaire et non irritant en irritation primaire cutanée, ce qui est un excellent résultat par rapport à un produit solaire classique. L'indice de protection solaire mesuré a été comparable à celui obtenu avec une émulsion classique contenant les mêmes filtres aux mêmes doses.

**B - Test d'activité comédolytique**

L'animal choisi pour le test d'activité comédolytique est la souris Hairless Rhino (hr rh), de sexe femelle, ce choix étant dû au fait que la peau d'un tel animal présente une grande densité de comédons de large diamètre et d'orifice étroit.

L'application d'agents comédolytiques sur la peau de l'animal provoque l'ouverture de l'orifice du comédon, la libération du matériel corné et du sébum qu'il contient.

Deux lots sont constitués, chacun des lots comportant 6 souris âgées de 6 semaines au début de l'essai et pesant en moyenne 18 grammes chacune.

Le premier lot est constitué de souris traitées avec de l'eau distillée (lot témoin négatif), le second lot est constitué de souris traitées avec le produit à étudier, un tel traitement consistant en une application topique du produit étudié sur la zone interscapsulaire à la dose de 0,02 ml, 5 jours sur 7 pendant 21 jours.

Les animaux sont sacrifiés à l'issue des trois semaines de traitement, 24 heures après la dernière application.

Des biopsies de la peau sont alors prélevées au niveau des zones traitées des animaux et à partir de ces biopsies, des coupes sont préparées, en vue d'une étude morphométrique classique réalisée selon les méthodes connues de l'homme du métier.

Les paramètres suivants sont mesurés :

diamètre de l'ouverture de comédon à la surface      soit d

diamètre du comédon      soit D

profil comédonien      soit R = d/D

Le rapport R = d/D permet de quantifier l'action des agents comédolytiques.

Le pourcentage d'inhibition des comédons est calculé pour le produit à étudier par rapport au témoin négatif, soit le rapport suivant :

$$\% \ inhibition = \frac{(R \ produit - R \ témoin \ négatif) \times 100}{R \ témoin \ négatif}$$

Le test d'activité kératolytique décrit ci-dessus a été réalisé pour le produit de l'exemple 5 et le produit de l'exemple 5 a été comparé à deux préparations renfermant également de la vitamine A, soit une émulsion eau-silicone et une émulsion à phase gémellaire préparées ainsi qu'il est indiqué ci-après.

On a ainsi obtenu pour le produit de l'exemple 5, 53 % de l'activité kératolytique de la vitamine A acide (Alberel gel) prise comme standard, alors qu'avec l'émulsion eau/silicone on obtenait 20 %, et avec l'émulsion à phase gémellaire 39 %.

Les résultats montrent bien l'avantage de la préparation selon l'invention, illustrée par l'exemple 5 sur les préparations classiques.

**Préparation des produits utilisés dans l'étude clinique**

**Emulsion à phases gémellaires**

On réalise l'émulsion suivante :

| | | |
|---|---|---|
| - alcool stéarylique | | 1,0 g |
| - alcool cétylique | | 2,0 g |
| - cétéaryl octanoate | | 4,0 g |
| - polysorbate 60 | | 4,0 g |
| - sorbitan stéarate | | 4,0 g |
| - caprylic caprique triglycérides | | 3,0 g |
| - beurre de karité | | 3,0 g |
| - acétate d'oleyle (anti lipase) | | 2,0 g |
| - huile de silicone | | 0,5 g |
| - tocophérols | | 0,05 g |
| - eau déminéralisée | QS | 100,0 g |
| - polymère carboxyvinylique | | 0,3 g |
| - conservateurs | | 0,7 g |
| - lubrajel MS | | 5,0 g |
| - palm. de vit. A 1,7 M UI/g | | 0,12 g |
| - hydroxyde de sodium 10 % | | 0,3 g |
| - parfum | | 0,2 g |
| - biolysat hafnia | | 0,02 g |

**Emulsion eau/silicones**

On réalise l'émulsion suivante à 60°C :

| | | |
|---|---|---|
| - eau déminéralisée | QS | 100,22 g |
| - chlorure de sodium | | 0,8 g |
| - acide citrique pur | | 0,01 g |
| - méthylparaben | | 0,25 g |
| - conservateurs | | 2,0 g |
| - biolysat hafnia | | 0,02 g |
| - stéarate d'isocétyle | | 3,0 g |
| - arlacel 83 | | 0,8 g |
| - huile de ricin hydrogénée | | 0,3 g |
| - elfacos ST 9 ® | | 2,0 g |
| - acétate d'oleyle (anti-lipase) | | 2,0 g |
| - propylparaben | | 0,15 g |
| - silicone DC 3225 ® | | 9,0 g |
| - silicone volatile | | 4,0 g |
| - bronopol | | 0,05 g |
| - palmitate de vit. A 1,7 M UI/g | | 0,12 g |
| - Parfum | | 0,3 g |

**Revendications**

1. Emulsion multiple se présentant sous la forme d'une émulsion eau/huile/eau constituée d'une phase dispersée qui est une émulsion du type eau/huile et d'un milieu de dispersion aqueux, caractérisée en ce

   que le milieu de dispersion aqueux est une phase aqueuse gélifiée et ne contient pas d'émulsionnant,
   que le gélifiant contenu dans la phase aqueuse de dispersion est constitué d'une solution à base de l'un ou de plusieurs composés choisis parmi le polyglycéryl méthacrylate, les carbomers et les carbomers modifiés, et que la quantité du gélifiant polyglycéryl méthacrylate est de 5 à 15 % de la composition totale.

2. Emulsion multiple selon la revendication 1, caractérisée en ce que un ou plusieurs principes actifs hydrosolubles sont incorporés dans la phase aqueuse de l'émulsion eau/huile et/ou dans la phase aqueuse de dispersion.

3. Emulsion multiple selon l'une des revendications 1 ou 2, caractérisée en ce que un ou plusieurs principes actifs liposolubles sont incorporés dans la phase grasse de l'émulsion eau/huile.

4. Emulsion multiple selon l'une des revendications 1 à 3, caractérisée en ce que les principes actifs hydrosolubles sont choisis parmi le lactate de sodium, des extraits de biolysat hafnia, des extraits de biolysat de Klebsillia pneumoniae et des filtres solaires hydrosolubles.

5. Emulsion multiple selon l'une des revendications 1 à 4, caractérisée en ce que les principes actifs liposolubles sont choisis parmi le palmitate de vitamine A, des filtres solaires liposolubles, des insaponifiables d'origine végétale, des huiles d'origine végétale, de l'acétate de tocophérols, des tocophérols naturels, du farnesol et des céramides naturelles ou de synthèse.

6. Emulsion multiple selon l'une des revendications 1 à 5, caractérisée en ce que les volumes des différentes phases sont tels que :

- la phase aqueuse interne représente 20 à 30 % de la composition totale,
- la phase grasse représente 10 à 20 % de la composition totale,
- et la phase aqueuse externe représente 50 à 60 % de la composition totale.

7. Procédé de préparation d'une émulsion multiple selon l'une des revendications 1 à 6, comprenant :

- la préparation de la phase dispersée en mélangeant, de préférence en chauffant, la phase aqueuse et la phase grasse sous forte agitation et
- le mélange de ladite phase dispersée avec la phase aqueuse externe, de préférence à température ordinaire, sous faible agitation jusqu'à formation de l'émulsion multiple,
procédé caractérisé en ce

que la phase aqueuse externe est gélifiée et ne contient pas d'émulsionnant, et
que le gélifiant que l'on utilise pour préparer la phase aqueuse externe est choisi parmi :

- une solution à base de polyglycéryl méthacrylate,
- des carbomers,
- des carbomers modifiés.

8. Composition cosmétique destinée en particulier à la protection solaire, caractérisée en ce qu'elle renferme une émulsion multiple selon l'une des revendications 1 à 6 et des excipients usuels dans l'émulsion multiple.

9. Composition cosmétique selon la revendication 8, caractérisée en ce qu'elle se présente sous forme d'une lotion solaire.

10. Composition cosmétique destinée aux peaux acnéiques, aux peaux déshydratées, aux peaux lésées, aux peaux ridées, caractérisée en ce qu'elle renferme une émulsion multiple selon l'une des revendications 1 à 6 et des excipients usuels.

11. Application d'émulsion multiple selon l'une des revendications 1 à 6, à la préparation de composition cosmétique telle que définie aux revendications 8 à 10.

## Claims

1. Multiple emulsion being presented in the form of a water/oil/water emulsion constituted by a dispersed phase which is an emulsion of water/oil type and an aqueous dispersion medium, characterized

in that the aqueous dispersion medium is a gelled aqueous phase and does not contain an emulsifier,
in that the gelling agent contained in the aqueous dispersion phase is constituted by a base solution of one or more compounds chosen from polyglyceryl methacrylate, carbomers and modified carbomers, and
in that the quantity of polyglyceryl methacrylate gelling agent is from 5 to 15% of the total composition.

2. Multiple emulsion according to claim 1, characterized in that one or more hydrosoluble active ingredients are incorporated in the aqueous phase of the water/oil emulsion and/or in the aqueous dispersion phase.

3. Multiple emulsion according to one of claims 1 or 2, characterized in that one or more liposoluble active ingredients are incorporated in the oily phase of the water/oil emulsion.

4. Multiple emulsion according to one of claims 1 to 3, characterized in that the hydrosoluble active ingredients are chosen from sodium lactate, extracts of hafnia biolysate, extracts of Klebsillia pneumoniae biolysate and hydrosoluble sunscreens.

5. Multiple emulsion according to one of claims 1 to 4, characterized in that the liposoluble active ingredients are chosen from the palmitate of vitamin A, liposoluble sunscreens, nonsaponifiable matter of vegetable origin, oils of vegetable origin, tocopherol acetates, natural tocopherols, farnesol and natural or synthetic ceramides.

6. Multiple emulsion according to one of claims 1 to 5, characterized in that the volumes of the different phases are such that:

- the internal aqueous phase represents 20 to 30% of the total composition,
- the oily phase represents 10 to 20% of the total composition,
- the external aqueous phase represents 50 to 60% of the total composition.

7. Preparation process for a multiple emulsion according to one of claims 1 to 6, including:

- preparation of the dispersed phase by mixing, preferably while heating, the aqueous phase and the oily phase under vigorous agitation and
- the mixture of said dispersed phase with the external aqueous phase, preferably at room temperature, under gentle agitation until the formation of the multiple emulsion, process characterized

  in that the aqueous phase is gelled and does not contain an emulsifier, and
  in that the gelling agent which is used to prepare the external aqueous phase is chosen from:

  - a solution based on polyglycerol methacrylate,
  - carbomers,
  - modified carbomers.

8. Cosmetic composition intended in particular for sun protection, characterized in that it contains a multiple emulsion according to one of claims 1 to 6 and the usual excipients for a multiple emulsion.

9. Cosmetic composition according to claim 8, characterized in that it is presented in the form of a sun lotion.

10. Cosmetic composition intended for skin which is prone to acne, very dry skin, damaged skin, wrinkled skin, characterized in that it contains a multiple emulsion according to one of claims 1 to 6 and the usual excipients.

11. Use of a multiple emulsion according to one of claims 1 to 6, for the preparation of a cosmetic composition as defined in claims 8 to 10.

**Patentansprüche**

1. Mehrfach-Emulsion in Form einer Wasser/Öl/Wasser-Emulsion, bestehend aus einer dispergierten Phase, die eine Emulsion vom Typ Wasser/Öl darstellt, und aus einem wäßrigen Dispersionsmilieu, dadurch gekennzeichnet, daß das wäßrige Dispersionsmilieu aus einer wäßrigen, gelierten Phase gebildet ist und keinen Emulgator enthält, daß weiterhin das in der wäßrigen Phase der Dispersion enthaltene Geliermittel aus einer Lösung auf der Grundlage einer oder mehrerer Verbindungen besteht, die unter Polyglycerylmethacrylat, den Carbomeren und den modifizierten Carbomeren ausgewählt sind, wobei der Mengenanteil des Geliermittels Polyglycerylmethacrylat 5 bis 15 % der Gesamtzusammensetzung ausmacht.

2. Mehrfach-Emulsion nach Anspruch 1, dadurch gekennzeichnet, daß einer oder mehrere wasserlösliche Wirkstoffe in die wäßrige Phase der Wasser/Öl-Emulsion und/oder in die wäßrige Dispersionsphase eingearbeitet sind.

3. Mehrfach-Emulsion nach einem der Ansprüche 1 oder 2, dadurch gekennzeichnet, daß ein oder mehrere fettlösliche Wirkstoffe in die Fettphase der Wasser/Öl-Emulsion eingearbeitet sind.

4. Mehrfach-Emulsion nach einem der Ansprüche 1 bis 3, dadurch gekennzeichnet, daß die wasserlöslichen Wirkstoffe aus Natriumlaktat, Biolysatextrakten aus Hafnia, Biolysatextrakten aus Klebsiella pneumoniae und wasserlöslichen Sonnenfiltersubstanzen ausgewählt sind.

5. Mehrfach-Emulsion nach einem der Ansprüche 1 bis 4, dadurch gekennzeichnet, daß die fettlöslichen Wirkstoffe aus dem Palmitat von Vitamin A, fettlöslichen Sonnenfiltersubstanzen und nicht verseifbaren Substanzen pflanzlichen Ursprungs, Ölen pflanzlichen Ursprungs, aus dem Acetat von Tocopherolen, natürlichen Tocopherolen, Farnesol und natürlichen oder synthetischen Ceramiden ausgewählt sind.

6. Mehrfach-Emulsion nach einem der Ansprüche 1 bis 5, dadurch gekennzeichnet, daß die Volumina der verschiedenen Phasen sind wie folgt:

- daß die interne wäßrige Phase 20 bis 30 % der Gesamtzusammensetzung,
- die Fettphase 10 bis 20 % der Gesamtzusammensetzung und
- die externe wäßrige Phase 50 bis 60 % der Gesamtzusammensetzung ausmachen.

7. Verfahren zur Herstellung einer Mehrfach-Emulsion nach einem der Ansprüche 1 bis 6 unter Einschluß der Maßnahmen:

- Herstellung der dispergierten Phase durch Vermischen, vorzugsweise unter Erwärmung der wäßrigen Phase und Fettphase unter starkem Rühren sowie
- das Vermischen dieser dispergierten Phase mit der externen wäßrigen Phase, vorzugsweise bei Normaltemperatur unter schwachem Rühren bis zur Bildung der Mehrfach-Emulsion, wobei das Verfahren dadurch gekennzeichnet ist, daß
- die externe wäßrige Phase geliert wird und keinen Emulgator enthält, wobei das verwendete Geliermittel zur Herstellung der externen wäßrigen Phase unter den folgenden ausgewählt wird:
- einer Lösung auf Basis von Polyglycerylmethacrylat,
- Carbomeren, sowie
- modifizierten Carbomeren.

8. Kosmetische Zubereitung, die insbesondere für den Schutz vor Sonne bestimmt ist, dadurch gekennzeichnet, daß sie eine Mehrfach-Emulsion nach einem der Ansprüche 1 bis 6 sowie Exzipientien, wie sie in einer Mehrfach-Emulsion üblich sind, umfaßt.

9. Kosmetische Zubereitung nach Anspruch 8, dadurch gekennzeichnet, daß sie in Form einer Sonnenlotion vorliegt.

10. Kosmetische Zubereitung, die für Aknehaut, dehydratisierte Haut, lädierte Haut und faltige Haut bestimmt ist, dadurch gekennzeichnet, daß sie eine Mehrfach-Emulsion nach einem der Ansprüche 1 bis 6 sowie übliche Exzipientien umfaßt.

11. Verwendung einer Mehrfach-Emulsion nach einem der Ansprüche 1 bis 6 für die Herstellung einer kosmetischen Zubereitung gemäß Definition in den Ansprüchen 8 bis 10.